# EUROPEAN PATENT SPECIFICATION

(11) **EP 4 185 104 B1**
(45) Date of publication and mention of the grant of the patent: **02.12.2026**
(21) Application number: 21846872.6
(22) Date of filing: 29.06.2021
(51) Int. Cl.: A01K 29/00, A61B 5/00, A61B 5/103, A61B 5/11, A63B 24/00, A61B 5/024, H04W 52/02, G06F 1/3203, G06F 1/3206, G04G 21/02, A01K 11/00

(54) **A SYSTEM AND METHOD FOR EFFICIENT ANIMAL MONITORING DEVICE POWER CONSUMPTION MANAGEMENT**
SYSTEM UND VERFAHREN ZUR EFFIZIENTEN STROMVERBRAUCHSVERWALTUNG EINER TIERÜBERWACHUNGSVORRICHTUNG
SYSTÈME ET PROCÉDÉ POUR LA GESTION EFFICACE DE LA CONSOMMATION D'ÉNERGIE D'UN DISPOSITIF DE SURVEILLANCE D'ANIMAUX

(30) Priority: 21.07.2020 IL 27620120
(43) Date of publication of application: 31.05.2023
(73) Proprietor: S.C.R. (Engineers) Limited, 4250553 Netanya (IL)
(72) Inventor: GENZEL, Eran, 4250553 Netanya (IL); WEINBERG, Eden, 4250553 Netanya (IL)
(74) Representative: Marks & Clerk LLP
(86) International application number: PCT/IL2021/050797
(87) International publication number: WO 2022/018714

(56) References cited:
- WO-A1-2006/036567
- WO-A1-2019/226100
- WO-A2-2019/040721
- US-A1- 2006 020 177
- US-A1- 2016 022 201
- US-A1- 2018 113 498
- US-A1- 2019 373 857
- US-A9- 2016 034 634

## Description

### TECHNICAL FIELD

The invention relates to power consumption management, and more specifically to efficient animal monitoring device power consumption management.

### BACKGROUND

Animal monitoring devices (such as animal monitoring tags, animal monitoring collars, or any other device that can monitor various parameters relating to an animal) are used to sense information on the animal's health status, often continuously sensing information, and optionally to transmit the obtained information wirelessly to an external server. Due to the fact that the animal monitoring devices are often attached to the animal's body (in order to be able to collect the required information), the animal monitoring devices are ideally as small and light-weight as possible in order reduce animal discomfort.

On the other hand, it is desirable to reduce maintenance requirements of the animal monitoring devices, which can be cumbersome. One type of maintenance includes making sure that a power source of the animal monitoring devices does not run out, or if it does - that it is replaced quickly in order to enable continuous health monitoring of the animal wearing the animal monitoring device. In many cases, in a single farm, animal monitoring devices are attached to a large number of animals (in some cases the numbers can be hundreds, thousands or tens of thousands of animals). Accordingly, it is desirable to have a power source that can provide power for long time-spans. However, usually the power capacity of the power source directly depends on its size and weight, so that the bigger and heavier it is - the higher its power capacity is. In addition, power source costs have a substantial effect on the costs of the animal monitoring devices, which preferably should be kept as low as possible in order to provide a cost-effective animal health monitoring solution.

Devices for monitoring activities of human beings are well-known. For example, US2006020177 discloses an apparatus and a method for measuring the quantity of exercise of a user by using an acceleration sensor. US2016022201 relates to automatic movement detection utilizing a wearable electronic device that can generate movement measures based on movement data generated by sensors of a wearable electronic device.

Devices for monitoring activities of animals are also well-known. For example, WO2019040721 relates to animal health tracking, and, in particular, to the tracking of physiological and/or behavioral parameters of multiple animals in an animal population.

However, there is a need to balance between the requirement of keeping the tags small and light-weight on the one hand (in order to reduce animal discomfort), and providing a long-lasting power source with high power capacity (in order to reduce power source related maintenance) at reasonable costs.

There is thus a need in the art for a new method and system for efficient animal monitoring device power consumption management.

### GENERAL DESCRIPTION

In accordance with a first aspect of the presently disclosed subject matter, there is provided a monitoring system, comprising: a sensing mechanism comprising one or more sensors configured to obtain information relating to a subject, the sensing mechanism having a plurality of operation modes, each of the operation modes having respective power consumption ranges; a power source capable of supplying power to the sensors in accordance with the power consumption ranges; and a processing circuitry configured to: cause activation of the sensing mechanism at a first operation mode of the operation modes to obtain a plurality of first readings from the sensors over a first period of time; analyze the first readings to determine a behavioral state of the subject, the behavioral state being one of a plurality of predetermined behavioral states, wherein (a) each behavioral state of the predetermined behavioral states is associated with a distinct group of a plurality of predetermined sub-behavioral states, and (b) the sub-behavioral states are different than the behavioral states; based on the behavioral state, cause activation of the sensing mechanism at a second operation mode of the operation modes to obtain a plurality of second readings from the sensors over a second period of time, the second operation mode having a higher power consumption range than the first operation mode; and analyze the second readings to determine a first sub-behavioral state of the subject, the first sub-behavioral state being one of the sub-behavioral states of the group of the predetermined sub-behavioral states associated with the behavioral state.

The processing circuitry is further configured to: cause, after determining the sub-behavioral state of the subject, activation of the sensing mechanism at the first operation mode of the operation modes to obtain a plurality of third readings from the sensors over a third period of time; analyze the third readings to determine a second behavioral state of the subject, the second behavioral state being one of the plurality of predetermined behavioral states; based on the second behavioral state, cause activation of the sensing mechanism at a third operation mode of the operation modes to obtain a plurality of fourth readings from the sensors over a fourth period of time, the third operation mode having a second higher power consumption range than the first operation mode; and analyze the fourth readings to determine a second sub-behavioral state of the subject, the second sub-behavioral state being one of the sub-behavioral states of the group of the predetermined sub-behavioral states associated with the second behavioral state.

Upon the second behavioral state being identical to the behavioral state, causing the activation of the sensing mechanism at the third operation mode is performed upon a time, between the determination of the second behavioral state and one of (a) the determination of the behavioral state or (b) the determination of the first sub-behavioral state, exceeding a threshold.

In some cases, the determination of at least one of (a) the behavioral state of the subject, or (b) the first sub-behavioral state, is also based on analysis of historical behavioral patterns associated with the subject.

In some cases, the operation modes define at least one of: a sampling rate of one or more of the sensors, a sensitivity of one or more of the sensors, a dynamic range of one or more of the sensors, an accuracy of one or more of the sensors, or a bandwidth of one or more of the sensors.

In some cases, the sensing mechanism, the power source and the processing circuitry are comprised within a tag attachable to the subject.

In some cases, the sensing mechanism and the power source are comprised within a tag attachable to the subject.

In some cases, the processing circuitry is part of a server, the tag further comprises a transceiver capable of transmitting the information to the server, and the processing circuitry is further configured to receive the first readings and the second readings from the tag utilizing the transmitter.

In some cases, the transceiver is a wireless transceiver.

In some cases, the sensors include one or more of the following: a vibration sensor, a temperature sensor, a velocity sensor, an acceleration sensor, a gyroscope, a magnetometer, a pedometer, a location sensor, a heart rate sensor, a moisture sensor.

In some cases, the subject is an animal.

In some cases, the power source is a battery.

In some cases, the information obtained by at least one of the sensors is physiological information obtained from the subject.

In some cases, the information obtained by at least one of the sensors is geo-spatial information.

In some cases, the information obtained by at least one of the sensors is environmental information.

In accordance with a second aspect of the presently disclosed subject matter, there is provided a monitoring method, comprising: causing, by a processing circuitry, activation of a sensing mechanism at a first operation mode of a plurality of operation modes of the sensing mechanism, to obtain a plurality of first readings from the sensors over a first period of time, wherein (a) the sensing mechanism comprising one or more sensors configured to obtain information relating to a subject, (b) each of the operation modes having respective power consumption ranges; analyzing, by the processing circuitry, the first readings to determine a behavioral state of the subject, the behavioral state being one of a plurality of predetermined behavioral states, wherein (a) each behavioral state of the predetermined behavioral states is associated with a distinct group of a plurality of predetermined sub-behavioral states, and (b) the sub-behavioral states are different than the behavioral states; based on the behavioral state, causing, by the processing circuitry, activation of the sensing mechanism at a second operation mode of the operation modes to obtain a plurality of second readings from the sensors over a second period of time, the second operation mode having a higher power consumption range than the first operation mode; and analyzing, by the processing circuitry, the second readings to determine a first sub-behavioral state of the subject, the first sub-behavioral state being one of the sub-behavioral states of the group of the predetermined sub-behavioral states associated with the behavioral state.

The further comprises: causing, by the processing circuitry, after determining the sub-behavioral state of the subject, activation of the sensing mechanism at the first operation mode of the operation modes to obtain a plurality of third readings from the sensors over a third period of time; analyzing, by the processing circuitry, the third readings to determine a second behavioral state of the subject, the second behavioral state being one of the plurality of predetermined behavioral states; based on the second behavioral state, causing, by the processing circuitry, activation of the sensing mechanism at a third operation mode of the operation modes to obtain a plurality of fourth readings from the sensors over a fourth period of time, the third operation mode having a second higher power consumption range than the first operation mode; and analyzing, by the processing circuitry, the fourth readings to determine a second sub-behavioral state of the subject, the second sub-behavioral state being one of the sub-behavioral states of the group of the predetermined sub-behavioral states associated with the second behavioral state.

Upon the second behavioral state being identical to the behavioral state, causing the activation of the sensing mechanism at the third operation mode is performed upon a time, between the determination of the second behavioral state and one of (a) the determination of the behavioral state or (b) the determination of the first sub-behavioral state, exceeding a threshold.

In some cases, the determination of at least one of (a) the behavioral state of the subject, or (b) the first sub-behavioral state, is also based on analysis of historical behavioral patterns associated with the subject.

In some cases, the operation modes define at least one of: a sampling rate of one or more of the sensors, a sensitivity of one or more of the sensors, a dynamic range of one or more of the sensors, an accuracy of one or more of the sensors, or a bandwidth of one or more of the sensors.

In some cases, the sensing mechanism, a power source capable of supplying power to the sensors in accordance with the power consumption ranges, and the processing circuitry are comprised within a tag attachable to the subject.

In some cases, the sensing mechanism and a power source capable of supplying power to the sensors in accordance with the power consumption ranges, are comprised within a tag attachable to the subject.

In some cases, the processing circuitry is part of a server, the tag further comprises a transceiver capable of transmitting the information to the server, and the method further comprises receiving the first readings and the second readings from the tag utilizing the transmitter.

In some cases, the transceiver is a wireless transceiver.

In some cases, the sensors include one or more of the following: a vibration sensor, a temperature sensor, a velocity sensor, an acceleration sensor, a gyroscope, a magnetometer, a pedometer, a location sensor, a heart rate sensor, a moisture sensor.

In some cases, the subject is an animal.

In some cases, the information obtained by at least one of the sensors is physiological information obtained from the subject.

In some cases, the information obtained by at least one of the sensors is geo-spatial information.

In some cases, the information obtained by at least one of the sensors is environmental information.

In accordance with a third aspect of the presently disclosed subject matter, there is provided a non-transitory computer readable storage medium having computer readable program code embodied therewith, the computer readable program code, executable by at least one processing circuitry of a computer to perform a method comprising: causing, by a processing circuitry, activation of a sensing mechanism at a first operation mode of a plurality of operation modes of the sensing mechanism, to obtain a plurality of first readings from the sensors over a first period of time, wherein (a) the sensing mechanism comprising one or more sensors configured to obtain information relating to a subject, (b) each of the operation modes having respective power consumption ranges; analyzing, by the processing circuitry, the first readings to determine a behavioral state of the subject, the behavioral state being one of a plurality of predetermined behavioral states, wherein (a) each behavioral state of the predetermined behavioral states is associated with a distinct group of a plurality of predetermined sub-behavioral states, and (b) the sub-behavioral states are different than the behavioral states; based on the behavioral state, causing, by the processing circuitry, activation of the sensing mechanism at a second operation mode of the operation modes to obtain a plurality of second readings from the sensors over a second period of time, the second operation mode having a higher power consumption range than the first operation mode; and analyzing, by the processing circuitry, the second readings to determine a first sub-behavioral state of the subject, the first sub-behavioral state being one of the sub-behavioral states of the group of the predetermined sub-behavioral states associated with the behavioral state.

In accordance with a fourth aspect of the presently disclosed subject matter, there is provided a monitoring system, comprising a processing circuitry configured to: provide historical information of historical behavioral patterns of one or more subjects at respective points in time, the historical behavioral patterns define expected behavioral states of the respective subjects at the respective points in time; obtain, from a sensing mechanism operating at a first operation mode of a plurality of operation modes, current information, the current information including one or more readings attributable to at least one of the subjects, being identified subjects; analyze the current information to determine current behavioral states of one or more of the identified subjects; and upon the current behavioral states of one or more of the identified subjects deviating from the historical behavioral patterns of the respective subjects, instruct the sensing mechanism to change an operation mode of the sensing mechanism to a second operation mode of the operation modes, other than the first operation mode.

In accordance with a fifth aspect of the presently disclosed subject matter, there is provided a monitoring method, comprising: providing, by a processing circuitry, historical information of historical behavioral patterns of one or more subjects at respective points in time, the historical behavioral patterns define expected behavioral states of the respective subjects at the respective points in time; obtaining, by the processing circuitry, from a sensing mechanism operating at a first operation mode of a plurality of operation modes, current information, the current information including one or more readings attributable to at least one of the subjects, being identified subjects; analyzing, by the processing circuitry, the current information to determine current behavioral states of one or more of the identified subjects; and upon the current behavioral states of one or more of the identified subjects deviating from the historical behavioral patterns of the respective subjects, instructing, by the processing circuitry, the sensing mechanism to change an operation mode of the sensing mechanism to a second operation mode of the operation modes, other than the first operation mode.

In accordance with a sixth aspect of the presently disclosed subject matter, there is provided a non-transitory computer readable storage medium having computer readable program code embodied therewith, the computer readable program code, executable by at least one processing circuitry of a computer to perform a method comprising: providing, by a processing circuitry, historical information of historical behavioral patterns of one or more subjects at respective points in time, the historical behavioral patterns define expected behavioral states of the respective subjects at the respective points in time; obtaining, by the processing circuitry, from a sensing mechanism operating at a first operation mode of a plurality of operation modes, current information, the current information including one or more readings attributable to at least one of the subjects, being identified subjects; analyzing, by the processing circuitry, the current information to determine current behavioral states of one or more of the identified subjects; and upon the current behavioral states of one or more of the identified subjects deviating from the historical behavioral patterns of the respective subjects, instructing, by the processing circuitry, the sensing mechanism to change an operation mode of the sensing mechanism to a second operation mode of the operation modes, other than the first operation mode.

### BRIEF DESCRIPTION OF THE DRAWINGS

In order to understand the presently disclosed subject matter and to see how it may be carried out in practice, the subject matter will now be described, by way of non-limiting examples only, with reference to the accompanying drawings, in which:
**Fig. 1** is a block diagram schematically illustrating one example of a monitoring system, in accordance with the presently disclosed subject matter;
**Fig. 2** is a flowchart illustrating one example of a sequence of operations carried out for management of power consumption based on behavioral states of the animal, in accordance with the presently disclosed subject matter;
**Fig. 3** is another flowchart illustrating one example of an additional sequence of operations carried out for management of power consumption based on behavioral states of the animal, in accordance with the presently disclosed subject matter;
**Fig. 4** is a schematic illustration of exemplary behavioral states and sub-behavioral states of an animal, based on which power consumption is managed, in accordance with the presently disclosed subject matter; and
**Fig. 5** is a flowchart illustrating another example of a sequence of operations carried out for management of power consumption based on historical behavioral patterns and current behavioral states of the animal, in accordance with the presently disclosed subject matter.

### DETAILED DESCRIPTION

In the following detailed description, numerous specific details are set forth in order to provide a thorough understanding of the presently disclosed subject matter. However, it will be understood by those skilled in the art that the presently disclosed subject matter may be practiced without these specific details. In other instances, well-known methods, procedures, and components have not been described in detail so as not to obscure the presently disclosed subject matter.

In the drawings and descriptions set forth, identical reference numerals indicate those components that are common to different embodiments or configurations.

Unless specifically stated otherwise, as apparent from the following discussions, it is appreciated that throughout the specification discussions utilizing terms such as "providing", "obtaining", "analyzing", "instructing", "causing" or the like, include action and/or processes of a computer that manipulate and/or transform data into other data, said data represented as physical quantities, e.g. such as electronic quantities, and/or said data representing the physical objects. The terms "computer", "processor", "processing circuitry" and "controller" should be expansively construed to cover any kind of electronic device with data processing capabilities, including, by way of non-limiting example, a personal desktop/laptop computer, a server, a computing system, a communication device, a smartphone, a tablet computer, a smart television, a processor (e.g. digital signal processor (DSP), a microcontroller, a field programmable gate array (FPGA), an application specific integrated circuit (ASIC), etc.), a group of multiple physical machines sharing performance of various tasks, virtual servers co-residing on a single physical machine, any other electronic computing device, and/or any combination thereof.

The operations in accordance with the teachings herein may be performed by a computer specially constructed for the desired purposes or by a general-purpose computer specially configured for the desired purpose by a computer program stored in a non-transitory computer readable storage medium. The term "non-transitory" is used herein to exclude transitory, propagating signals, but to otherwise include any volatile or non-volatile computer memory technology suitable to the application.

As used herein, the phrase "for example," "such as", "for instance" and variants thereof describe non-limiting embodiments of the presently disclosed subject matter. Reference in the specification to "one case", "some cases", "other cases" or variants thereof means that a particular feature, structure or characteristic described in connection with the embodiment(s) is included in at least one embodiment of the presently disclosed subject matter. Thus, the appearance of the phrase "one case", "some cases", "other cases" or variants thereof does not necessarily refer to the same embodiment(s).

It is appreciated that, unless specifically stated otherwise, certain features of the presently disclosed subject matter, which are, for clarity, described in the context of separate embodiments, may also be provided in combination in a single embodiment. Conversely, various features of the presently disclosed subject matter, which are, for brevity, described in the context of a single embodiment, may also be provided separately or in any suitable sub-combination.

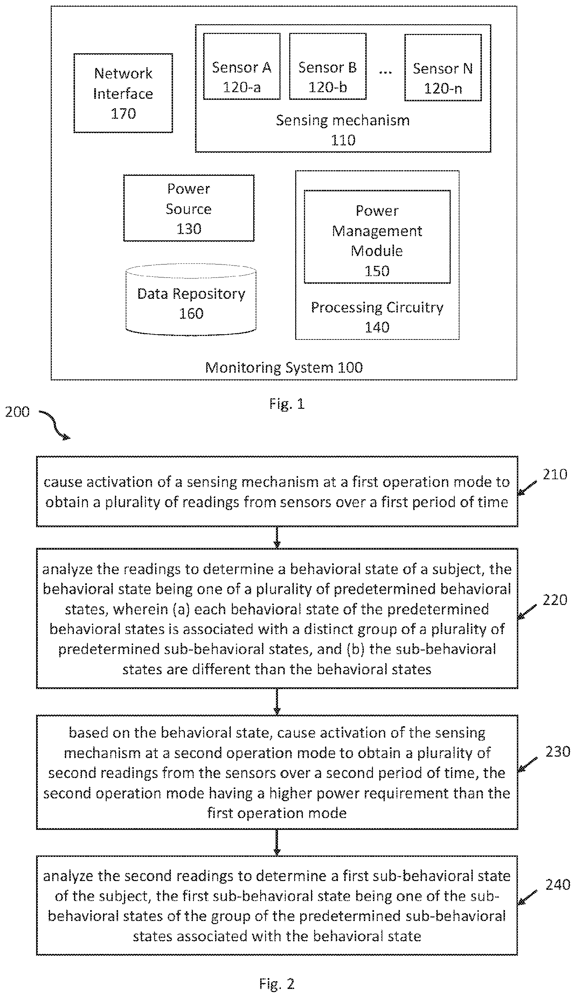

In embodiments of the presently disclosed subject matter, fewer, more and/or different stages than those shown in **Figs. 2****,** **3** **and** **5** may be executed. In embodiments of the presently disclosed subject matter one or more stages illustrated in **Figs. 2****,** **3** **and** **5** may be executed in a different order and/or one or more groups of stages may be executed simultaneously. **Fig. 1** illustrates a general schematic of the system architecture in accordance with an embodiment of the presently disclosed subject matter. Each module in **Fig. 1** can be made up of any combination of software, hardware and/or firmware that performs the functions as defined and explained herein. The modules in **Fig. 1** may be centralized in one location or dispersed over more than one location, as detailed herein. In other embodiments of the presently disclosed subject matter, the system may comprise fewer, more, and/or different modules than those shown in **Fig. 1****.**

Any reference in the specification to a method should be applied mutatis mutandis to a system capable of executing the method and should be applied mutatis mutandis to a non-transitory computer readable medium that stores instructions that once executed by a computer result in the execution of the method.

Any reference in the specification to a system should be applied mutatis mutandis to a method that may be executed by the system and should be applied mutatis mutandis to a non-transitory computer readable medium that stores instructions that may be executed by the system.

Any reference in the specification to a non-transitory computer readable medium should be applied mutatis mutandis to a system capable of executing the instructions stored in the non-transitory computer readable medium and should be applied mutatis mutandis to method that may be executed by a computer that reads the instructions stored in the non-transitory computer readable medium.

Bearing this in mind, attention is drawn to **Fig. 1****,** a block diagram schematically illustrating one example of a monitoring system, in accordance with the presently disclosed subject matter.

According to the presently disclosed subject matter, monitoring system 100 comprises a power source 130. The power source 130 can be, for example, a battery that can optionally be rechargeable. However, power source 130 can alternatively be any other source of energy that can provide power for enabling operation of one or more components of the monitoring system 100.

Monitoring system 100 further includes a sensing mechanism 110, which includes one or more sensors (sensor A 120-a, sensor B 120-b, ..., sensor N 120-n, where n is a natural number) configured to collect information relating to a subject, such as an animal (e.g. cattle, pets, fish, swine, poultry, livestock, etc.).

The information collected by the sensors (sensor A 120-a, sensor B 120-b, ..., sensor N 120-n - collectively referred to herein as "sensors) can include, for example: (a) physiological information collected from the animal's body, such as its temperature, heart rate, biometric information, velocity, acceleration (optionally directional acceleration), spatial orientation, etc., (b) environmental information about the environment of the animal, such as the ambient temperature, ambient moisture, etc., (c) geo-spatial information such as animal's geographical location, relative location, etc. Accordingly, the sensors can include one or more of the following: a vibration sensor, a temperature sensor, a velocity sensor, an acceleration sensor (e.g. an accelerometer), a gyroscope, a magnetometer, a pedometer, a location sensor (e.g. a Global Positioning System receiver), a heart rate sensor, a moisture sensor, etc.

The sensing mechanism 110 has a plurality of operation modes, each having respective power consumption ranges and an average/median power consumption. Each operation mode defines at least one sensor operation parameter that affects the sensor's power consumption ranges and/or its average/median power consumption (and thus, the sensing mechanism's 110 power consumption range and/or average/median power consumption): a sampling rate of one or more of the sensing mechanism's 110 sensors (noting that the sensor's sampling rate and the sensor's power consumption are positively correlated so that the higher the sensor's sampling rate is - the more power it consumes), a sensitivity of one or more of the sensing mechanism's 110 sensors (noting that the sensor's sensitivity and the sensor's power consumption are positively correlated so that the higher the sensor's sensitivity is - the more power it consumes), a dynamic range of one or more of the sensing mechanism's 110 sensors (noting that the sensor's dynamic range and the sensor's power consumption are positively correlated so that the broader the sensor's dynamic range is - the more power it consumes), an accuracy of one or more of the sensing mechanism's 110 sensors (noting that the sensor's accuracy and the sensor's power consumption are positively correlated so that the higher the sensor's accuracy is - the more power it consumes), or a bandwidth of one or more of the sensing mechanism's 110 sensors (noting that the sensor's bandwidth and the sensor's power consumption are positively correlated so that the broader the sensor's bandwidth is - the more power it consumes). It is to be noted that in some cases other and/or additional sensor operation parameters can also have an effect on the sensor's power consumption range and/or the sensor's average/median power consumption.

Each operation mode results in a respective power consumption range and average/median power consumption, and at least a first operation mode of the operation modes has a different power consumption range and/or a different average/median power consumption, than a second operation mode of the operation modes.

The sensing mechanism 110 receives power from the power source 130, to meet its power requirements, which are affected by the sensing mechanism's 110 operation mode. It is to be noted that a first operation mode can result in a power consumption that is higher than a second operation mode, and in such case, the power source 130 is required to provide, on average, more power to the sensing mechanism 110 when operating in the first operation mode in comparison to the second operation mode. Clearly, the more power consumed by the sensing mechanism, the shorter the power supply 130 life (the time-span during which the power supply 130 can supply power to the sensing mechanism).

Monitoring system 100 further comprises a processing circuitry 140. Processing circuitry 140 can be one or more processing units (e.g. central processing units), microprocessors, microcontrollers (e.g. microcontroller units (MCUs)) or any other computing devices or modules, including multiple and/or parallel and/or distributed processing units, which are adapted to independently or cooperatively process data for controlling relevant monitoring system 110 resources and for enabling operations related to monitoring system's 110 resources.

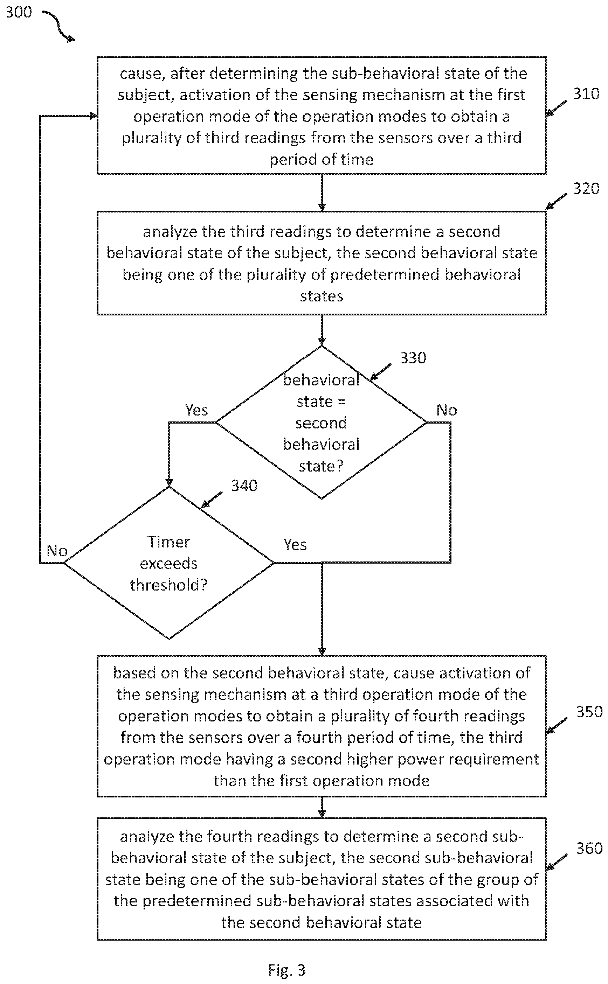

Processing circuitry 140 comprises a power management module 150, configured to control the sensing mechanism's 110 operation modes, as further detailed herein, inter alia with reference to Figs. 2 and 3, in order to enable efficient power consumption thereof.

Monitoring system 100 can further comprise, or be otherwise associated with, a data repository 160 (e.g. a database, a storage system, a memory including Read Only Memory - ROM, Random Access Memory - RAM, or any other type of memory, etc.) configured to store data, optionally including, inter alia, information of operation modes of the sensing mechanism 110, information enabling determination of behavioral and sub-behavioral states of a subject (as will be detailed herein inter alia with respect to Figs. 2-4) from which the sensors collect information, indications of minimal periods of time before switching between operation modes in certain cases, historical behavioral patterns of a subject from which the sensors collect information, etc. Data repository 160 can be further configured to enable retrieval and/or update and/or deletion of the stored data. It is to be noted that in some cases, data repository 160 can be distributed, while the monitoring system 100 has access to the information stored thereon, e.g. via a wired or wireless network to which monitoring system 100 is able to connect.

Having described the various components of the monitoring system 100, it is to be noted that in some cases, the sensing mechanism 110, the power source 130 and the processing circuitry 140 are comprised within a device attachable to a subject (such as an animal monitoring tag, an animal monitoring collar, etc.) and the processing circuitry 140 can control the operation modes of the sensing mechanism 110 without intervention of an external entity (e.g. a server). However, in other cases, the sensing mechanism and the power source are comprised within a device attachable to the subject (such as an animal monitoring tag, an animal monitoring collar, etc.), whereas the processing circuitry 140 is part of a server external to the device. Such server can service a plurality of devices, each comprising its own sensing mechanism 110 and power source 130, and each attached to a respective subject. In those cases where the processing circuitry 140 is external to the device (e.g. animal monitoring tag/collar, etc.), the device can comprise a controller that can control the operation of the sensing mechanism 110, and a transceiver (e.g. a wireless transceiver), or any other network interface 170, that enables sending the information collected by the sensors to the server, and receiving control commands from the server for controlling the sensing mechanism's 110 operation modes.

Attention is now drawn to Fig. 2, a flowchart illustrating one example of a sequence of operations carried out for management of power consumption based on behavioral states of the animal, in accordance with the presently disclosed subject matter.

According to certain examples of the presently disclosed subject matter, monitoring system 100 can be configured to perform a power consumption management process 200-A, e.g. utilizing the power management module 150.

As indicated herein, the sensing mechanism 110 has a plurality of operation modes, each having respective power consumption ranges and/or average/median power consumption (e.g. due to differences in operation parameters of the sensors comprised within the sensing mechanism 110). Monitoring system 100 is configured to cause (by the processing resource 140 directly, or indirectly, e.g. by the processing resource 140 utilizing a controller that can be comprised within a device such as a tag/collar) activation of the sensing mechanism 110, at a first operation mode of the operation modes to obtain a plurality of first readings from the sensors over a first period of time (e.g. 15 seconds, 30 seconds, 45 seconds, 60 seconds, etc.) (**block 210**). The first operation mode can be an operation mode that has low power consumption range relatively to the other operation modes (e.g., at least part of its power consumption range is below the power consumption ranges of the other operation modes). Additionally, or alternatively, the first operation mode can be an operation mode that has a lower average/median power consumption relatively to the other operation modes.

Monitoring system 100 is further configured to analyze the first readings to determine a behavioral state of the subject (**block 220**). The determined behavioral state is one of a plurality of predetermined behavioral states of interest, each associated with a distinct group of a plurality of predetermined sub-behavioral states, while the sub-behavioral states are different than the behavioral states. It is to be noted that the difference between the behavioral states and the sub-behavioral states can be a difference in the granularity or a difference of degree, and not necessarily completely different behaviors. For example, a behavioral state can be "walking", whereas the respective sub-behavioral states can be "walking slow", "walking fast", "walking sideways" and "laming". "Walking slow", "walking fast", "walking sideways" and "laming" are all types of walking, but the behavioral state "walking" and the sub-behavioral states "walking slow", "walking fast", "walking sideways" and "laming" are considered different.

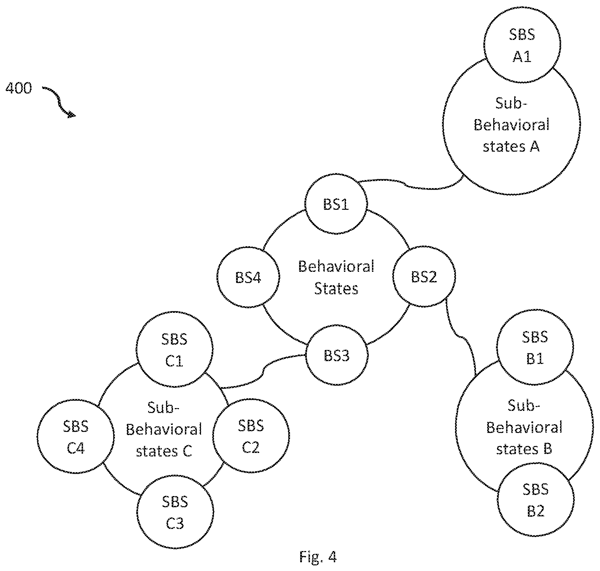

Attention is drawn in this respect to Fig. 4, a schematic illustration of exemplary behavioral states and sub-behavioral states of an animal, based on which power consumption is managed, in accordance with the presently disclosed subject matter.

The illustration shows, by way of example, a group of four possible behavioral states: BS1, BS2, BS3 and BS4, part of which are associated with a distinct group of sub-behavioral states. BS1 is associated with sub-behavioral states A which include sub-behavioral state (SBS) A1; BS2 is associated with sub-behavioral states B which include SBS B1 and SBS B2; BS3 is associated with sub-behavioral states C which include SBS C1, SBS C2, SBS C3 and SBS C4; and BS4 is not associated with any sub-behavioral states.

It is to be noted that (a) each group of sub-behavioral states (sub-behavioral states A, sub-behavioral states B, sub-behavioral states C, and sub-behavioral states D) is different than the other groups of sub-behavioral states, and (b) each group of sub-behavioral states (sub-behavioral states A, sub-behavioral states B, sub-behavioral states C, and sub-behavioral states D) is different than the group of behavioral states (BS1, BS2, BS3 and BS4.

Returning to Fig. 2, it is to be noted that in some cases, the determination of the behavioral state made in block 220 is also based on analysis of historical behavioral patterns associated with the subject (and optionally behavioral patterns, and/or historical behavioral patterns, associated with other subjects, optionally of the same type). In view of the fact that the determination can optionally be probabilistic, using historical information of past behavioral patterns of the subject (or similar subjects), indicating how it (or they) behaved at different points-in-time in the past, can enable the monitoring system 100 to more accurately determine the subject's current behavioral state.

It is to be noted, in this respect, that: (a) utilization of historical behavioral pattern associated with the subject can enable identifying outliers in the behavior of the subject with respect to an expected behavior of the specific subject; (b) utilization of historical behavioral patterns associated with other subjects (optionally of the same type, e.g. when the subject is a cow - other cows), enable identifying outliers in the behavior of the subject with respect to an expected behavior of the other subjects; and (c) utilization of an expected behavioral pattern (e.g. of a hypothetical subject) can enable identifying outliers in the behavior of the subject with respect to the expected behavioral pattern. In such cases, outlier detection can provide a more accurate behavioral state determination regime, and behaviors that are outliers can be subject to more scrutiny examination before positive determination of the behavioral state.

After the determination of the behavioral state, and based on the determined behavioral state, monitoring system 100 causes activation of the sensing mechanism at a second operation mode of the operation modes to obtain a plurality of second readings from the sensors over a second period of time, noting that the second operation mode can have a higher power consumption range than the first operation mode (i.e. at least part of its power consumption range is above the power consumption ranges of the first operation mode), and/or the second operation mode can be an operation mode that has a higher average/median power consumption relatively to the first operation mode. (**block 230**). Accordingly, when a certain behavioral state of interest was identified, it is desirable to change an operation parameter of one or more of the sensors of the sensing mechanism 110 in order to obtain, for example, more information and/or more refined information and/or more granular information and/or more accurate information, to enable determination of a sub-behavioral state of the subject.

Monitoring system 100 analyzes the second readings to determine a first sub-behavioral state of the subject, the first sub-behavioral state being one of the sub-behavioral states of the group of the predetermined sub-behavioral states associated with the behavioral state (**block 240**).

It is to be noted that in some cases, similarly to the determination of the behavioral states, the determination of the sub-behavioral state made in block 240 is also based on analysis of historical behavioral patterns associated with the subject (and optionally behavioral patterns and/or historical behavioral patterns associated with other subjects, optionally of the same type). In view of the fact that the determination can optionally be probabilistic, using historical information of past behavioral patterns of the subject (or similar subjects), indicating how it (or they) behaved at different points-in-time in the past, can enable the monitoring system 100 to more accurately determine the subject's current sub-behavioral state.

Looking at an example, when the monitoring system 100 started its operation, the subject (e.g. a cow), was standing still. The monitoring system 100 activated the sensing mechanism 110 at a first operation mode, having a relatively low power consumption range and/or average/median power consumption. The monitoring system 100 analyzed the information collected by the sensing mechanism, 110 and at a certain point-in-time the results of the analysis show that the cow started walking (i.e. the monitoring system 100 determined that a behavioral state of the cow is "walking"). Upon making the determination that the cow is walking, the monitoring system 100 activated the sensing mechanism 110 at a second operation mode, having higher power consumption range and/or a higher average/median power consumption than the first operation mode. This may be required due to the fact that in order to determine a type of walking of the cow, more information and/or more refined information and/or more granular information and/or more accurate information, is required. The monitoring system 100 analyzed the information collected by the sensing mechanism 110 operating at the second operation mode, and at a certain point-in-time the results of the analysis show that the cow is "laming" (i.e. the monitoring system 100 determined that a sub-behavioral state of the cow is "laming").

It is to be further noted that in some cases, during performance of block 240 the second readings (or some of the second readings) are also analyzed to verify that the determined behavioral state of the subject did not change during the attempt to determine the first sub-behavioral state of the subject. This reevaluation can be based on analysis of all of the second readings (that may be obtained at a faster rate than the first readings due to the change on the sensing mechanism's operation mode), or only on some of the second readings, e.g. according to the rate of obtainment of the readings analyzed for a similar purpose on block 220. In case the behavioral state of the subject changed during the attempt to determine the first sub-behavioral state of the subject, the monitoring system 100 can return to block 230 and cause activation the sensing mechanism at an operation mode suitable for the newly determined behavioral state.

It is to be noted, with reference to Fig. 2, that some of the blocks can be integrated into a consolidated block or can be broken down to a few blocks and/or other blocks may be added. It should be also noted that whilst the flow diagram is described also with reference to the system elements that realizes them, this is by no means binding, and the blocks can be performed by elements other than those described herein.

Turning to **Fig. 3****,** there is shown another flowchart illustrating one example of an additional sequence of operations carried out for management of power consumption based on behavioral states of the animal, in accordance with the presently disclosed subject matter.

According to certain examples of the presently disclosed subject matter, monitoring system 100 can be configured to continue the power consumption management process 200-A shown in Fig. 2 with power consumption management process 200-B shown in Fig. 3, e.g. utilizing the power management module 150.

For this purpose, monitoring system 100 causes, after determining the sub-behavioral state of the subject at block 240, activation of the sensing mechanism at the first operation mode of the operation modes to obtain a plurality of readings from the sensors over a period of time (**block 310**). It is to be noted that upon determination of the sub-behavioral state of the subject, it is no longer required to activate the sensing mechanism at a relatively high power consuming operation mode, so returning to the first operation mode, that has a lower power consumption range and/or a lower average/median power consumption than the second operation mode, servers the purpose of power consumption saving.

Monitoring system 100 analyzes the readings obtained at block 310 to determine a second behavioral state of the subject, the second behavioral state being one of the plurality of predetermined behavioral states referred to at block 220 (**block 320**).

It is to be noted that in some cases, the determination of the behavioral state made in block 320 is also based on analysis of historical behavioral patterns associated with the subject (and optionally behavioral patterns and/or historical behavioral patterns, associated with other subjects, optionally of the same type). In view of the fact that the determination can optionally be probabilistic, using historical information of past behavioral patterns of the subject (or similar subjects), indicating how it (or they) behaved at different points-in-time in the past, can enable more accurate determination of the subject's current behavioral state.

It is to be noted, in this respect, that: (a) utilization of historical behavioral pattern associated with the subject can enable identifying outliers in the behavior of the subject with respect to an expected behavior of the specific subject; (b) utilization of historical behavioral patterns associated with other subjects (optionally of the same type, e.g. when the subject is a cow - other cows), enable identifying outliers in the behavior of the subject with respect to an expected behavior of the other subjects; and (c) utilization of an expected behavioral pattern (e.g. of a hypothetical subject) can enable identifying outliers in the behavior of the subject with respect to the expected behavioral pattern. In such cases, outlier detection can provide a more accurate behavioral state determination regime, and behaviors that are outliers can be subject to more scrutiny examination before positive determination of the behavioral state.

At this stage, the monitoring system 100 can be configured to perform a check to see if the second behavioral state determined at block 320 is the same behavioral state as the one determined at block 220 (**block 330**). If the second behavioral state is the same behavioral state as the one determined at block 220, it may be desirable to avoid activating the sensing mechanism at an operation mode having higher power consumption range and/or a higher average/median power consumption than the first operation mode for a certain time-period (optionally a pre-determined time-period or a dynamic time-period calculated based on one or more parameters), e.g. due to the fact that the probability that the subject's sub-behavioral state changed during such time period is low. For example, when determining that a certain animal is lame, there may be no value in redetermining that it is lame during the next 24 hours, hence power consumption can be reduced due to preventing reactivating the sensing mechanism at a relatively high power-consuming operation mode for making such lameness determination. Accordingly, monitoring system 100 can be configured to check if a timer initiated in dependence (e.g. immediately upon, at a certain time before/after, etc.) to (a) the determination of the behavioral sate at block 220 or (b) the determination of the sub-behavioral state at block 240, exceeded a threshold (**block 340**). If the timer does not exceed a threshold, the monitoring system 100 returns to block 310 and continues to analyze the readings obtained from the sensors, while the operation mode of the sensing mechanism 110 remains unchanged (it does not switch to a higher power consuming operation mode as it would have been without said timing mechanism).

If the timer exceeds the threshold, or if the second behavioral state determined at block 320 is not the same behavioral state as the one determined at block 220, monitoring system 100 causes, based on the second behavioral state, activation of the sensing mechanism at another operation mode of the operation modes, having a higher power consumption range and/or a higher average/median power consumption than the first operation mode, to obtain a plurality of readings from the sensors over another period of time (**block 350**). As indicated herein, when a certain behavioral state of interest was identified, it is desirable to change an operation parameter of one or more of the sensors of the sensing mechanism 110 in order to obtain, for example, more information and/or more refined information and/or more granular information and/or more accurate information, to enable determination of a sub-behavioral state of the subject.

Monitoring system 100 analyzes the readings obtained at block 350 to determine a sub-behavioral state of the subject, the sub-behavioral state being one of the sub-behavioral states of the group of the predetermined sub-behavioral states associated with the behavioral state determined at block 320 (**block 360**).

It is to be noted that in some cases, the determination of the sub-behavioral state made in block 340 is also based on analysis of historical behavioral patterns associated with the subject (and optionally behavioral patterns, and/or historical behavioral patterns, associated with other subjects, optionally of the same type). In view of the fact that the determination can optionally be probabilistic, using historical information of past behavioral patterns of the subject (or similar subjects), indicating how it (or they) behaved at different points-in-time in the past, can enable the monitoring system 100 to more accurately determine the subject's current sub-behavioral state.

It is to be noted, in this respect, that: (a) utilization of historical behavioral pattern associated with the subject can enable identifying outliers in the behavior of the subject with respect to an expected behavior of the specific subject; (b) utilization of historical behavioral patterns associated with other subjects (optionally of the same type, e.g. when the subject is a cow - other cows), enable identifying outliers in the behavior of the subject with respect to an expected behavior of the other subjects; and (c) utilization of an expected behavioral pattern (e.g. of a hypothetical subject) can enable identifying outliers in the behavior of the subject with respect to the expected behavioral pattern. In such cases, outlier detection can provide a more accurate behavioral state determination regime, and behaviors that are outliers can be subject to more scrutiny examination before positive determination of the behavioral state.

Continuing the example provided with reference to Fig. 2, after the monitoring system determined that the cow is "laming" (i.e. the monitoring system 100 determined that a sub-behavioral state of the cow is "laming"), the monitoring system 100 returns the sensing mechanism 110 to operate at the first operation mode, having a relatively low power consumption range and/or a relatively low average/median power consumption. The monitoring system 100 analyzed the information collected by the sensing mechanism, 110 and immediately identified that the cow is walking (i.e. the monitoring system 100 determined that a behavioral state of the cow is "walking"). However, the monitoring system 100 just finalized an analysis of the sub-behavior state of the cow and identified that it is "laming", and there is no point in reactivating the sensing mechanism at a higher power consuming operation mode, because that would probably lead to the same determination that the cow is laming, while wasting the power supply's 130 resources unnecessarily. Accordingly, the monitoring system 100 does not switch to a higher power consuming operation mode for a certain time-period upon the determined behavioral state remaining unchanged with reference to a precedingly determined behavioral state of the cow. However, if a certain time-period (e.g. one or more minutes/hours/days) did pass the monitoring system 100 activated the sensing mechanism 110 at another operation mode, having higher power consumption range and/or higher average/median power consumption than the first operation mode.

If the monitoring system 100 identified that the cow is breathing heavily instead of walking (i.e. the monitoring system 100 determined that a behavioral state of the cow is "heavy breathing"), the monitoring system 100 activated the sensing mechanism 110 at another operation mode, having higher power consumption range and/or higher average/median power consumption than the first operation mode, irrespective of any time-related considerations (assuming that any time-related considerations associated with the "heavy breathing" behavioral state do not prevent doing so). This may be required due to the fact that in order to determine a type of heavy breathing of the cow, more information and/or more refined information and/or more granular information and/or more accurate information, is required. The monitoring system 100 analyzed the information collected by the sensing mechanism 110 operating at the second operation mode, and at a certain point-in-time the results of the analysis show that the cow is "coughing" (i.e. the monitoring system 100 determined that a sub-behavioral state of the cow is "cough").

From that point, the monitoring system 100 can return to block 310 and the process can continuously repeat itself.

It is to be noted, with reference to Fig. 3, that some of the blocks can be integrated into a consolidated block or can be broken down to a few blocks and/or other blocks may be added. It should be also noted that whilst the flow diagram is described also with reference to the system elements that realizes them, this is by no means binding, and the blocks can be performed by elements other than those described herein.

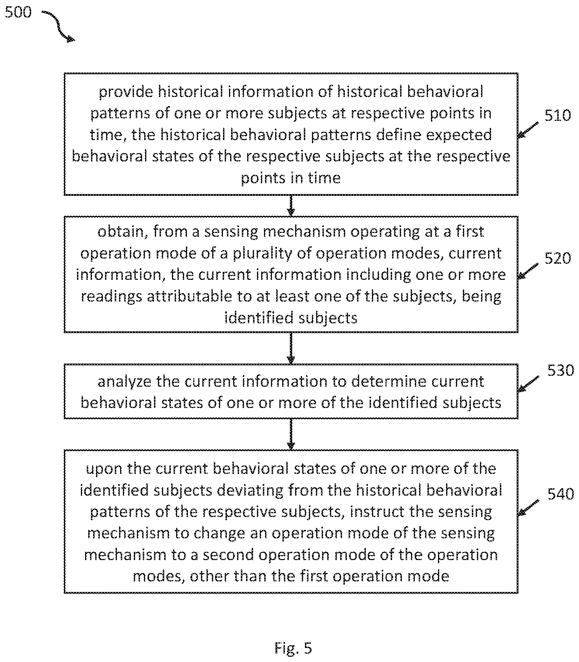

Attention is now drawn to **Fig. 5****,** a flowchart illustrating another example of a sequence of operations carried out for management of power consumption based on historical behavioral patterns and current behavioral states of the animal, in accordance with the presently disclosed subject matter.

According to certain examples of the presently disclosed subject matter, monitoring system 100 can be configured to perform a history-based power consumption management process 500, e.g. utilizing the power management module 150.

For this purpose, monitoring system 100 can be configured to provide historical information of historical behavioral patterns of one or more subjects at respective points in time (e.g. during the day, the year, the month, or any other time-frame), the historical behavioral patterns define expected behavioral states of the respective subjects at the respective points in time (**block 510**). For example, the historical behavioral patterns can show that a certain subject falls asleep in over 90% of the days between 20:00-21:00 and wakes up between 4:00-5:00. Accordingly, it is expected that such subject will falls asleep between 20:00-21:00 and wake up between 4:00-5:00. Similarly, the historical behavioral patterns can show that all subjects of a certain type eat during certain constant time windows along the day. Accordingly, it is expected that each subject will follow the same pattern and eat during such time windows on a daily basis.

Monitoring system 100 is further configured to obtain, from the sensing mechanism operating at a first operation mode of a plurality of operation modes, current information, the current information including one or more readings attributable to at least one of the subjects, being identified subjects (**block 520**). The first operation mode can be an operation mode that has low power consumption range and/or low average/median power consumption relatively to the other operation modes.

Monitoring system 100 can analyze the current information to determine current behavioral states of one or more of the identified subjects (**block 530**). The determination of the behavioral state can be made similarly to the determination described with reference to blocks 220 and 320.

Based upon the current behavioral states of one or more of the identified subjects deviating from the historical behavioral patterns of the respective subjects, monitoring system 100 can instruct the sensing mechanism 110 to change its operation mode to a second operation mode of the operation modes having higher power consumption ranges and/or higher average/median power consumptions than the first operation mode, in order to obtain more information and/or more refined information and/or more granular information and/or more accurate information, in order to explain the discrepancy between the subject's expected behavior and its actual behavior.

It is to be noted, with reference to Fig. 5, that some of the blocks can be integrated into a consolidated block or can be broken down to a few blocks and/or other blocks may be added. It should be also noted that whilst the flow diagram is described also with reference to the system elements that realizes them, this is by no means binding, and the blocks can be performed by elements other than those described herein.

It is to be understood that the presently disclosed subject matter is not limited in its application to the details set forth in the description contained herein or illustrated in the drawings. The presently disclosed subject matter is capable of other embodiments and of being practiced and carried out in various ways. Hence, it is to be understood that the phraseology and terminology employed herein are for the purpose of description and should not be regarded as limiting. The invention is set out in the appended claims.

It will also be understood that the system according to the presently disclosed subject matter can be implemented, at least partly, as a suitably programmed computer. Likewise, the presently disclosed subject matter contemplates a computer program being readable by a computer for executing the disclosed methods. The presently disclosed subject matter further contemplates a machine-readable memory tangibly embodying a program of instructions executable by the machine for executing the disclosed methods.

## Claims

1. A monitoring system (100), comprising:
a sensing mechanism (110) comprising one or more sensors configured to obtain information relating to a subject, the sensing mechanism (110) having a plurality of operation modes, each of the operation modes having respective power consumption ranges;
a power source (130) capable of supplying power to the one or more sensors in accordance with the power consumption ranges; and
a processing circuitry (140) configured to:
cause activation of the sensing mechanism (110) at a first operation mode of the operation modes to obtain a plurality of first readings from the one or more sensors over a first period of time;
analyze the first readings to determine a behavioral state of the subject, the behavioral state being one of a plurality of predetermined behavioral states, wherein (a) each behavioral state of the predetermined behavioral states is associated with a distinct group of a plurality of predetermined sub-behavioral states, and (b) in at least one of the plurality of predetermined behavioral states at least part of said plurality of predetermined sub-behavioral states are degrees of said behavioral state;
based on the behavioral state, cause activation of the sensing mechanism (110) at a second operation mode of the operation modes to obtain a plurality of second readings from the one or more sensors over a second period of time, the second operation mode having a higher power consumption range than the first operation mode;
analyze the second readings to determine a first sub-behavioral state of the subject, the first sub-behavioral state being one of the sub-behavioral states of the group of the predetermined sub-behavioral states associated with the behavioral state;
cause, after determining the sub-behavioral state of the subject, activation of the sensing mechanism (110) at the first operation mode of the operation modes to obtain a plurality of third readings from the one or more sensors over a third period of time;
analyze the third readings to determine a second behavioral state of the subject, the second behavioral state being one of the plurality of predetermined behavioral states;
based on the second behavioral state, cause activation of the sensing mechanism (110) at a third operation mode of the operation modes to obtain a plurality of fourth readings from the one or more sensors over a fourth period of time, the third operation mode having a second higher power consumption range than the first operation mode, wherein upon the second behavioral state being identical to the behavioral state, causing the activation of the sensing mechanism (110) at the third operation mode is performed upon a time, between the determination of the second behavioral state and one of (a) the determination of the behavioral state or (b) the determination of the first sub-behavioral state, exceeding a threshold; and
analyze the fourth readings to determine a second sub-behavioral state of the subject, the second sub-behavioral state being one of the sub-behavioral states of the group of the predetermined sub-behavioral states associated with the second behavioral state.

2. A monitoring method, comprising:
causing, by a processing circuitry (140), activation of a sensing mechanism (110) comprising one or more sensors configured to obtain information relating to a subject at a first operation mode of a plurality of operation modes of the sensing mechanism (110), to obtain a plurality of first readings from the one or more sensors over a first period of time, wherein each of the operation modes having respective power consumption ranges;
analyzing, by the processing circuitry (140), the first readings to determine a behavioral state of the subject, the behavioral state being one of a plurality of predetermined behavioral states, wherein (a) each behavioral state of the predetermined behavioral states is associated with a distinct group of a plurality of predetermined sub-behavioral states, and (b) in at least one of the plurality of predetermined behavioral states at least part of said plurality of predetermined sub-behavioral states are degrees of said behavioral state;
based on the behavioral state, causing, by the processing circuitry (140), activation of the sensing mechanism (110) at a second operation mode of the operation modes to obtain a plurality of second readings from the one or more sensors over a second period of time, the second operation mode having a higher power consumption range than the first operation mode;
analyzing, by the processing circuitry (140), the second readings to determine a first sub-behavioral state of the subject, the first sub-behavioral state being one of the sub-behavioral states of the group of the predetermined sub-behavioral states associated with the behavioral state;
causing, by the processing circuitry (140), after determining the sub-behavioral state of the subject, activation of the sensing mechanism (110) at the first operation mode of the operation modes to obtain a plurality of third readings from the one or more sensors over a third period of time;
analyzing, by the processing circuitry (140), the third readings to determine a second behavioral state of the subject, the second behavioral state being one of the plurality of predetermined behavioral states;
based on the second behavioral state, causing, by the processing circuitry (140), activation of the sensing mechanism (110) at a third operation mode of the operation modes to obtain a plurality of fourth readings from the one or more sensors over a fourth period of time, the third operation mode having a second higher power consumption range than the first operation mode, wherein upon the second behavioral state being identical to the behavioral state, causing the activation of the sensing mechanism (110) at the third operation mode is performed upon a time, between the determination of the second behavioral state and one of (a) the determination of the behavioral state or (b) the determination of the first sub-behavioral state, exceeding a threshold; and
analyzing, by the processing circuitry (140), the fourth readings to determine a second sub-behavioral state of the subject, the second sub-behavioral state being one of the sub-behavioral states of the group of the predetermined sub-behavioral states associated with the second behavioral state.

3. The monitoring system (100) of claim 1, or the monitoring method of claim 2, wherein the determination of at least one of (a) the behavioral state of the subject, or (b) the first sub-behavioral state, is also based on analysis of historical behavioral patterns associated with the subject.

4. The monitoring system (100) of claim 1, or the monitoring method of claim 2, wherein the operation modes define at least one of: a sampling rate of one or more of the one or more sensors, a sensitivity of one or more of the one or more sensors, a dynamic range of one or more of the one or more sensors, an accuracy of one or more of the one or more sensors, or a bandwidth of one or more of the one or more sensors.

5. The monitoring system (100) of claim 1, or the monitoring method of claim 2, wherein the sensing mechanism (110), a power source (130) capable of supplying power to the one or more sensors in accordance with the power consumption ranges, and the processing circuitry (140) are comprised within a tag attachable to the subject.

6. The monitoring system (100) of claim 1, or the monitoring method of claim 2, wherein the sensing mechanism (110) and a power source (130) capable of supplying power to the one or more sensors in accordance with the power consumption ranges, are comprised within a tag attachable to the subject.

7. The monitoring system (100) of claim 6, or the monitoring method of claim 6, wherein the processing circuitry (140) is part of a server, the tag further comprises a transceiver capable of transmitting the information to the server, and the method further comprises receiving the first readings and the second readings from the tag utilizing a transmitter.

8. The monitoring system (100) of claim 1, or the monitoring method of claim 2, wherein the one or more sensors include one or more of the following: a vibration sensor, a temperature sensor, a velocity sensor, an acceleration sensor, a gyroscope, a magnetometer, a pedometer, a location sensor, a heart rate sensor, a moisture sensor.

9. The monitoring system (100) of claim 1, or the monitoring method of claim 2, wherein the subject is an animal.

10. The monitoring system (100) of claim 1, or the monitoring method of claim 2, wherein the information obtained by at least one of the one or more sensors includes one or more of: (a) physiological information obtained from the subject, (b) geo-spatial information, or (c) environmental information.

11. A non-transitory computer readable storage medium having computer readable program code embodied therewith, the computer readable program code, executable by at least one processing circuitry (140) of a computer to perform a method comprising:
causing, by the processing circuitry (140), activation of a sensing mechanism (110) comprising one or more sensors configured to obtain information relating to a subject at a first operation mode of a plurality of operation modes of the sensing mechanism (110), to obtain a plurality of first readings from the sensors over a first period of time, wherein each of the operation modes having respective power consumption ranges;
analyzing, by the processing circuitry (140), the first readings to determine a behavioral state of the subject, the behavioral state being one of a plurality of predetermined behavioral states, wherein (a) each behavioral state of the predetermined behavioral states is associated with a distinct group of a plurality of predetermined sub-behavioral states, and (b) in at least one of the plurality of predetermined behavioral states at least part of said plurality of predetermined sub-behavioral states are degrees of said behavioral state;
based on the behavioral state, causing, by the processing circuitry (140), activation of the sensing mechanism (110) at a second operation mode of the operation modes to obtain a plurality of second readings from the one or more sensors over a second period of time, the second operation mode having a higher power consumption range than the first operation mode;
analyzing, by the processing circuitry (140), the second readings to determine a first sub-behavioral state of the subject, the first sub-behavioral state being one of the sub-behavioral states of the group of the predetermined sub-behavioral states associated with the behavioral state;
causing, by the processing circuitry (140), after determining the sub-behavioral state of the subject, activation of the sensing mechanism (110) at the first operation mode of the operation modes to obtain a plurality of third readings from the one or more sensors over a third period of time;
analyzing, by the processing circuitry (140), the third readings to determine a second behavioral state of the subject, the second behavioral state being one of the plurality of predetermined behavioral states;
based on the second behavioral state, causing, by the processing circuitry (140), activation of the sensing mechanism (110) at a third operation mode of the operation modes to obtain a plurality of fourth readings from the one or more sensors over a fourth period of time, the third operation mode having a second higher power consumption range than the first operation mode, wherein upon the second behavioral state being identical to the behavioral state, causing the activation of the sensing mechanism (110) at the third operation mode is performed upon a time, between the determination of the second behavioral state and one of (a) the determination of the behavioral state or (b) the determination of the first sub-behavioral state, exceeding a threshold; and
analyzing, by the processing circuitry (140), the fourth readings to determine a second sub-behavioral state of the subject, the second sub-behavioral state being one of the sub-behavioral states of the group of the predetermined sub-behavioral states associated with the second behavioral state.

## Patentansprüche

1. Überwachungssystem (100), umfassend:
einen Sensormechanismus (110) umfassend einen oder mehrere Sensoren, die konfiguriert sind, um Informationen über ein Subjekt zu erhalten, wobei der Sensormechanismus (110) über eine Vielzahl von Betriebsmodi verfügt, wobei jeder der Betriebsmodi einen jeweiligen Stromverbrauchsbereich aufweist;
eine Leistungsquelle (130), die in der Lage ist, den einen oder die mehreren Sensoren entsprechend den Stromverbrauchsbereichen mit Strom zu versorgen; und
eine Verarbeitungsschaltung (140), die konfiguriert ist zum:
Bewirken der Aktivierung des Sensormechanismus (110) in einem ersten Betriebsmodus der Betriebsmodi, um eine Vielzahl von ersten Messwerten von dem einen oder den mehreren Sensoren über einen ersten Zeitraum zu erhalten;
Analysieren der ersten Messwerte, um einen Verhaltenszustand des Subjekts zu bestimmen, wobei der Verhaltenszustand einer von einer Vielzahl von vorbestimmten Verhaltenszuständen ist, wobei (a) jeder Verhaltenszustand der vorbestimmten Verhaltenszustände einer bestimmten Gruppe aus einer Vielzahl von vorbestimmten Unterverhaltenszuständen zugeordnet ist und (b) in mindestens einem der Vielzahl von vorbestimmten Verhaltenszuständen mindestens ein Teil dieser Vielzahl von vorbestimmten Unterverhaltenszuständen Abstufungen dieses Verhaltenszustands sind;
basierend auf dem Verhaltenszustand, Bewirken der Aktivierung des Sensormechanismus (110) in einem zweiten Betriebsmodus der Betriebsmodi, um eine Vielzahl von zweiten Messwerten von dem einem oder den mehreren Sensoren über einen zweiten Zeitraum zu erhalten, wobei der zweite Betriebsmodus einen höheren Stromverbrauchsbereich als der erste Betriebsmodus aufweist;
Analysieren der zweiten Messwerte, um einen ersten Unterverhaltenszustand des Subjekts zu bestimmen, wobei der erste Unterverhaltenszustand einer der Unterverhaltenszustände aus der Gruppe der vorbestimmten Unterverhaltenszustände ist, die dem Verhaltenszustand zugeordnet sind;
Bewirken, nach dem Bestimmen des Unterverhaltenszustands des Subjekts, der Aktivierung des Sensormechanismus (110) in dem ersten Betriebsmodus der Betriebsmodi, um eine Vielzahl von dritten Messwerten von dem einem oder den mehreren Sensoren über einen dritten Zeitraum zu erhalten;
Analysieren der dritten Messwerte, um einen zweiten Verhaltenszustand des Subjekts zu bestimmen, wobei der zweite Verhaltenszustand einer aus der Vielzahl von vorbestimmten Verhaltenszuständen ist;
basierend auf dem zweiten Verhaltenszustand, Bewirken der Aktivierung des Sensormechanismus (110) in einem dritten Betriebsmodus der Betriebsmodi, um eine Vielzahl von vierten Messwerten von dem einen oder den mehreren Sensoren über einen vierten Zeitraum zu erhalten, wobei der dritte Betriebsmodus einen zweiten höheren Stromverbrauchsbereich als der erste Betriebsmodus aufweist, wobei, wenn der zweite Verhaltenszustand identisch mit dem Verhaltenszustand ist, die Aktivierung des Sensormechanismus (110) in dem dritten Betriebsmodus zu einem Zeitpunkt erfolgt, der zwischen der Bestimmung des zweiten Verhaltenszustands und entweder (a) der Bestimmung des Verhaltenszustands oder (b) der Bestimmung des ersten Unterverhaltenszustands liegt und einen Schwellenwert überschreitet; und
Analysieren der vierten Messwerte, um einen zweiten Unterverhaltenszustand des Subjekts zu bestimmen, wobei der zweite Unterverhaltenszustand einer der Unterverhaltenszustände aus der Gruppe der vorbestimmten Unterverhaltenszustände ist, die dem zweiten Verhaltenszustand zugeordnet sind.

2. Überwachungsverfahren, umfassend:
Bewirken, durch eine Verarbeitungsschaltung (140), der Aktivierung eines Sensormechanismus (110) umfassend einen oder mehrere Sensoren, die konfiguriert sind, um Informationen über ein Subjekt zu erhalten, in einem ersten Betriebsmodus einer Vielzahl von Betriebsmodi des Sensormechanismus (110), um eine Vielzahl von ersten Messwerten von dem einen oder den mehreren Sensoren über einen ersten Zeitraum zu erhalten, wobei jeder der Betriebsmodi einen jeweiligen Stromverbrauchsbereich aufweist;
Analysieren, durch die Verarbeitungsschaltung (140), der ersten Messwerte, um einen Verhaltenszustand des Subjekts zu bestimmen, wobei der Verhaltenszustand einer von einer Vielzahl von vorbestimmten Verhaltenszuständen ist, wobei (a) jeder Verhaltenszustand der vorbestimmten Verhaltenszustände einer bestimmten Gruppe aus einer Vielzahl von vorbestimmten Unterverhaltenszuständen zugeordnet ist und (b) in mindestens einem der Vielzahl von vorbestimmten Verhaltenszuständen mindestens ein Teil dieser Vielzahl von vorbestimmten Unterverhaltenszuständen Abstufungen dieses Verhaltenszustands sind;
basierend auf dem Verhaltenszustand, Bewirken, durch die Verarbeitungsschaltung (140), der Aktivierung des Sensormechanismus (110) in einem zweiten Betriebsmodus der Betriebsmodi, um eine Vielzahl von zweiten Messwerten von dem einem oder den mehreren Sensoren über einen zweiten Zeitraum zu erhalten, wobei der zweite Betriebsmodus einen höheren Stromverbrauchsbereich als der erste Betriebsmodus aufweist;
Analysieren, durch die Verarbeitungsschaltung (140), der zweiten Messwerte, um einen ersten Unterverhaltenszustand des Subjekts zu bestimmen, wobei der erste Unterverhaltenszustand einer der Unterverhaltenszustände aus der Gruppe der vorbestimmten Unterverhaltenszustände ist, die dem Verhaltenszustand zugeordnet sind;
Bewirken, durch die Verarbeitungsschaltung (140), nach dem Bestimmen des Unterverhaltenszustands des Subjekts, der Aktivierung des Sensormechanismus (110) in dem ersten Betriebsmodus der Betriebsmodi, um eine Vielzahl von dritten Messwerten von dem einem oder den mehreren Sensoren über einen dritten Zeitraum zu erhalten;
Analysieren, durch die Verarbeitungsschaltung (140), der dritten Messwerte, um einen zweiten Verhaltenszustand des Subjekts zu bestimmen, wobei der zweite Verhaltenszustand einer aus der Vielzahl von vorbestimmten Verhaltenszuständen ist;
basierend auf dem zweiten Verhaltenszustand, Bewirken, durch die Verarbeitungsschaltung (140), der Aktivierung des Sensormechanismus (110) in einem dritten Betriebsmodus der Betriebsmodi, um eine Vielzahl von vierten Messwerten von dem einen oder den mehreren Sensoren über einen vierten Zeitraum zu erhalten, wobei der dritte Betriebsmodus einen zweiten höheren Stromverbrauchsbereich als der erste Betriebsmodus aufweist, wobei, wenn der zweite Verhaltenszustand identisch mit dem Verhaltenszustand ist, die Aktivierung des Sensormechanismus (110) in dem dritten Betriebsmodus zu einem Zeitpunkt erfolgt, der zwischen der Bestimmung des zweiten Verhaltenszustands und entweder (a) der Bestimmung des Verhaltenszustands oder (b) der Bestimmung des ersten Unterverhaltenszustands liegt und einen Schwellenwert überschreitet; und
Analysieren, durch die Verarbeitungsschaltung (140), der vierten Messwerte, um einen zweiten Unterverhaltenszustand des Subjekts zu bestimmen, wobei der zweite Unterverhaltenszustand einer der Unterverhaltenszustände aus der Gruppe der vorbestimmten Unterverhaltenszustände ist, die dem zweiten Verhaltenszustand zugeordnet sind.

3. Überwachungssystem (100) nach Anspruch 1 oder Überwachungsverfahren nach Anspruch 2, wobei die Bestimmung mindestens eines von (a) dem Verhaltenszustand des Subjekts oder (b) dem ersten Unterverhaltenszustand auch auf der Analyse historischer Verhaltensmuster des Subjekts basiert.

4. Überwachungssystem (100) nach Anspruch 1 oder Überwachungsverfahren nach Anspruch 2, wobei die Betriebsmodi mindestens eines von Folgendem definieren: eine Abtastrate eines oder mehrerer des einen oder der mehreren Sensoren, eine Empfindlichkeit eines oder mehrerer des einen oder der mehreren Sensoren, einen Dynamikbereich eines oder mehrerer des einen oder der mehreren Sensoren, eine Genauigkeit eines oder mehrerer des einen oder der mehreren Sensoren oder eine Bandbreite eines oder mehrerer des einen oder der mehreren Sensoren.

5. Überwachungssystem (100) nach Anspruch 1 oder Überwachungsverfahren nach Anspruch 2, wobei der Sensormechanismus (110), eine Leistungsquelle (130), die in der Lage ist, den einen oder die mehreren Sensoren entsprechend den Stromverbrauchsbereichen mit Strom zu versorgen, und die Verarbeitungsschaltung (140) in einem an dem Subjekt anbringbaren Tag untergebracht sind.

6. Überwachungssystem (100) nach Anspruch 1 oder Überwachungsverfahren nach Anspruch 2, wobei der Sensormechanismus (110) und eine Leistungsquelle (130), die in der Lage ist, den einen oder die mehreren Sensoren entsprechend den Stromverbrauchsbereichen mit Strom zu versorgen, in einem an dem Subjekt anbringbaren Tag untergebracht sind.

7. Überwachungssystem (100) nach Anspruch 6 oder Überwachungsverfahren nach Anspruch 6, wobei die Verarbeitungsschaltung (140) Teil eines Servers ist, das Tag ferner einen Transceiver umfasst, der in der Lage ist, die Informationen an den Server zu übertragen, und das Verfahren ferner das Empfangen der ersten und der zweiten Messwerte von dem Tag mittels eines Senders umfasst.

8. Überwachungssystem (100) nach Anspruch 1 oder Überwachungsverfahren nach Anspruch 2, wobei der eine oder die mehreren Sensoren eines oder mehrere von Folgendem einschließen: einen Vibrationssensor, einen Temperatursensor, einen Geschwindigkeitssensor, einen Beschleunigungssensor, ein Gyroskop, ein Magnetometer, einen Schrittzähler, einen Ortungssensor, einen Herzfrequenzsensor, einen Feuchtigkeitssensor.

9. Überwachungssystem (100) nach Anspruch 1 oder Überwachungsverfahren nach Anspruch 2, wobei das Subjekt ein Tier ist.

10. Überwachungssystem (100) nach Anspruch 1 oder Überwachungsverfahren nach Anspruch 2, wobei die durch mindestens einen von dem einen oder den mehreren Sensoren erhaltenen Informationen eines oder mehrere von Folgendem einschließen: (a) physiologische Informationen, die vom Subjekt gewonnen werden, (b) Geodaten oder (c) Umweltdaten.

11. Nichtflüchtiges, computerlesbares Speichermedium mit darin enthaltenem computerlesbarem Programmcode, wobei der computerlesbare Programmcode durch mindestens eine Verarbeitungseinheit (140) eines Computers ausgeführt werden kann, um ein Verfahren durchzuführen, das Folgendes umfasst:
Bewirken, durch die Verarbeitungsschaltung (140), der Aktivierung eines Sensormechanismus (110) umfassend einen oder mehrere Sensoren, die konfiguriert sind, um Informationen über ein Subjekt zu erhalten, in einem ersten Betriebsmodus einer Vielzahl von Betriebsmodi des Sensormechanismus (110), um eine Vielzahl von ersten Messwerten von den Sensoren über einen ersten Zeitraum zu erhalten, wobei jeder der Betriebsmodi einen jeweiligen Stromverbrauchsbereich aufweist;
Analysieren, durch die Verarbeitungsschaltung (140), der ersten Messwerte, um einen Verhaltenszustand des Subjekts zu bestimmen, wobei der Verhaltenszustand einer von einer Vielzahl von vorbestimmten Verhaltenszuständen ist, wobei (a) jeder Verhaltenszustand der vorbestimmten Verhaltenszustände einer bestimmten Gruppe aus einer Vielzahl von vorbestimmten Unterverhaltenszuständen zugeordnet ist und (b) in mindestens einem der Vielzahl von vorbestimmten Verhaltenszuständen mindestens ein Teil dieser Vielzahl von vorbestimmten Unterverhaltenszuständen Abstufungen dieses Verhaltenszustands sind;
basierend auf dem Verhaltenszustand, Bewirken, durch die Verarbeitungsschaltung (140), der Aktivierung des Sensormechanismus (110) in einem zweiten Betriebsmodus der Betriebsmodi, um eine Vielzahl von zweiten Messwerten von dem einem oder den mehreren Sensoren über einen zweiten Zeitraum zu erhalten, wobei der zweite Betriebsmodus einen höheren Stromverbrauchsbereich als der erste Betriebsmodus aufweist;
Analysieren, durch die Verarbeitungsschaltung (140), der zweiten Messwerte, um einen ersten Unterverhaltenszustand des Subjekts zu bestimmen, wobei der erste Unterverhaltenszustand einer der Unterverhaltenszustände aus der Gruppe der vorbestimmten Unterverhaltenszustände ist, die dem Verhaltenszustand zugeordnet sind;
Bewirken, durch die Verarbeitungsschaltung (140), nach dem Bestimmen des Unterverhaltenszustands des Subjekts, der Aktivierung des Sensormechanismus (110) in dem ersten Betriebsmodus der Betriebsmodi, um eine Vielzahl von dritten Messwerten von dem einem oder den mehreren Sensoren über einen dritten Zeitraum zu erhalten;
Analysieren, durch die Verarbeitungsschaltung (140), der dritten Messwerte, um einen zweiten Verhaltenszustand des Subjekts zu bestimmen, wobei der zweite Verhaltenszustand einer aus der Vielzahl von vorbestimmten Verhaltenszuständen ist;
basierend auf dem zweiten Verhaltenszustand, Bewirken, durch die Verarbeitungsschaltung (140), der Aktivierung des Sensormechanismus (110) in einem dritten Betriebsmodus der Betriebsmodi, um eine Vielzahl von vierten Messwerten von dem einen oder den mehreren Sensoren über einen vierten Zeitraum zu erhalten, wobei der dritte Betriebsmodus einen zweiten höheren Stromverbrauchsbereich als der erste Betriebsmodus aufweist, wobei, wenn der zweite Verhaltenszustand identisch mit dem Verhaltenszustand ist, die Aktivierung des Sensormechanismus (110) in dem dritten Betriebsmodus zu einem Zeitpunkt erfolgt, der zwischen der Bestimmung des zweiten Verhaltenszustands und entweder (a) der Bestimmung des Verhaltenszustands oder (b) der Bestimmung des ersten Unterverhaltenszustands liegt und einen Schwellenwert überschreitet; und
Analysieren, durch die Verarbeitungsschaltung (140), der vierten Messwerte, um einen zweiten Unterverhaltenszustand des Subjekts zu bestimmen, wobei der zweite Unterverhaltenszustand einer der Unterverhaltenszustände aus der Gruppe der vorbestimmten Unterverhaltenszustände ist, die dem zweiten Verhaltenszustand zugeordnet sind.

## Revendications

1. Système de surveillance (100) comprenant :
un mécanisme de détection (110) comprenant un ou plusieurs capteurs configurés pour obtenir des informations relatives à un sujet, le mécanisme de détection (110) présentant une pluralité de modes de fonctionnement, chacun des modes de fonctionnement présentant des plages de consommation d'énergie respectives ;
une source d'énergie (130) capable de fournir de l'énergie à l'un ou aux plusieurs capteurs conformément aux plages de consommation d'énergie ; et
un circuit de traitement (140) configuré pour :
provoquer une activation du mécanisme de détection (110) dans un premier mode de fonctionnement des modes de fonctionnement pour obtenir une pluralité de premières mesures à partir des un ou plusieurs capteurs sur une première période de temps ;
analyser les premières mesures pour déterminer un état comportemental du sujet, l'état comportemental étant l'un d'une pluralité d'états comportementaux prédéterminés, dans lequel (a) chaque état comportemental des états comportementaux prédéterminés est associé à un groupe distinct d'une pluralité d'états sous-comportementaux prédéterminés, et (b) dans au moins un de la pluralité d'états comportementaux prédéterminés, au moins une partie de ladite pluralité d'états sous-comportementaux prédéterminés sont des degrés dudit état comportemental ;
sur la base de l'état comportemental, provoquer l'activation du mécanisme de détection (110) dans un deuxième mode de fonctionnement des modes de fonctionnement pour obtenir une pluralité de deuxièmes mesures à partir des un ou plusieurs capteurs sur une deuxième période de temps, le deuxième mode de fonctionnement présentant une plage de consommation d'énergie plus élevée que le premier mode de fonctionnement ;
analyser les deuxièmes mesures pour déterminer un premier état sous-comportemental du sujet, le premier état sous-comportemental étant l'un des états sous-comportementaux du groupe des états sous-comportementaux prédéterminés associés à l'état comportemental ;
provoquer, après avoir déterminé l'état sous-comportemental du sujet, l'activation du mécanisme de détection (110) dans le premier mode de fonctionnement des modes de fonctionnement pour obtenir une pluralité de troisièmes mesures à partir des un ou plusieurs capteurs sur une troisième période de temps ;
analyser les troisièmes mesures pour déterminer un deuxième état comportemental du sujet, le deuxième état comportemental étant l'un de la pluralité d'états comportementaux prédéterminés ;
sur la base du deuxième état comportemental, provoquer l'activation du mécanisme de détection (110) dans un troisième mode de fonctionnement des modes de fonctionnement afin d'obtenir une pluralité de quatrièmes mesures à partir des un ou plusieurs capteurs sur une quatrième période de temps, le troisième mode de fonctionnement présentant une plage de consommation d'énergie supérieure à celle du premier mode de fonctionnement, dans lequel si le deuxième état comportemental est identique à l'état comportemental, l'activation du mécanisme de détection (110) dans le troisième mode de fonctionnement est effectuée lorsqu'un temps écoulé entre la détermination du deuxième état comportemental et une parmi (a) la détermination de l'état comportemental ou (b) la détermination du premier état sous-comportemental dépasse un seuil ; et
analyser les quatrièmes mesures pour déterminer un deuxième état sous-comportemental du sujet, le deuxième état sous-comportemental étant l'un des états sous-comportementaux du groupe des états sous-comportementaux prédéterminés associés au deuxième état comportemental.

2. Procédé de surveillance, comprenant les étapes consistant à :
provoquer, par un circuit de traitement (140), l'activation d'un mécanisme de détection (110) comprenant un ou plusieurs capteurs configurés pour obtenir des informations relatives à un sujet dans un premier mode de fonctionnement d'une pluralité de modes de fonctionnement du mécanisme de détection (110), pour obtenir une pluralité de premières mesures à partir des un ou plusieurs capteurs sur une première période de temps, dans lequel chacun des modes de fonctionnement présente des plages de consommation d'énergie respectives ;
analyser, par le circuit de traitement (140), les premières mesures pour déterminer un état comportemental du sujet, l'état comportemental étant l'un d'une pluralité d'états comportementaux prédéterminés, dans lequel (a) chaque état comportemental des états comportementaux prédéterminés est associé à un groupe distinct d'une pluralité d'états sous-comportementaux prédéterminés, et (b) dans au moins un de la pluralité d'états comportementaux prédéterminés, au moins une partie de ladite pluralité d'états sous-comportementaux prédéterminés sont des degrés dudit état comportemental ;
sur la base de l'état comportemental, provoquer l'activation, par le circuit de traitement (140), du mécanisme de détection (110) dans un deuxième mode de fonctionnement des modes de fonctionnement pour obtenir une pluralité de deuxièmes mesures à partir des un ou plusieurs capteurs sur une deuxième période de temps, le deuxième mode de fonctionnement présentant une plage de consommation d'énergie plus élevée que le premier mode de fonctionnement ;
analyser, par le circuit de traitement (140), les deuxièmes mesures pour déterminer un premier état sous-comportemental du sujet, le premier état sous-comportemental étant l'un des états sous-comportementaux du groupe des états sous-comportementaux prédéterminés associés à l'état comportemental ;
provoquer, par le circuit de traitement (140), après avoir déterminé l'état sous-comportemental du sujet, l'activation du mécanisme de détection (110) dans le premier mode de fonctionnement des modes de fonctionnement pour obtenir une pluralité de troisièmes mesures à partir des un ou plusieurs capteurs sur une troisième période de temps ;
analyser, par le circuit de traitement (140), les troisièmes mesures pour déterminer un deuxième état comportemental du sujet, le deuxième état comportemental étant l'un de la pluralité d'états comportementaux prédéterminés ;
sur la base du deuxième état comportemental, provoquer, par le circuit de traitement (140), l'activation du mécanisme de détection (110) dans un troisième mode de fonctionnement des modes de fonctionnement afin d'obtenir une pluralité de quatrièmes mesures à partir des un ou plusieurs capteurs sur une quatrième période de temps, le troisième mode de fonctionnement présentant une plage de consommation d'énergie supérieure à celle du premier mode de fonctionnement, dans lequel si le deuxième état comportemental est identique à l'état comportemental, l'activation du mécanisme de détection (110) dans le troisième mode de fonctionnement est effectuée lorsqu'un temps écoulé entre la détermination du deuxième état comportemental et une parmi (a) la détermination de l'état comportemental ou (b) la détermination du premier état sous-comportemental dépasse un seuil ; et
analyser, par le circuit de traitement (140), les quatrièmes mesures pour déterminer un deuxième état sous-comportemental du sujet, le deuxième état sous-comportemental étant l'un des états sous-comportementaux du groupe des états sous-comportementaux prédéterminés associés au deuxième état comportemental.

3. Système de surveillance (100) selon la revendication 1, ou procédé de surveillance selon la revendication 2, dans lequel la détermination d'au moins un parmi (a) l'état comportemental du sujet, ou (b) le premier état sous-comportemental, est également basée sur l'analyse des motifs comportementaux historiques associés au sujet.

4. Système de surveillance (100) selon la revendication 1, ou procédé de surveillance selon la revendication 2, dans lequel les modes de fonctionnement définissent au moins une parmi : une fréquence d'échantillonnage d'un ou plusieurs capteurs, une sensibilité d'un ou plusieurs capteurs, une plage dynamique d'un ou plusieurs capteurs, une précision d'un ou plusieurs capteurs, ou une bande passante d'un ou plusieurs capteurs.

5. Système de surveillance (100) selon la revendication 1, ou procédé de surveillance selon la revendication 2, dans lequel le mécanisme de détection (110), une source d'énergie (130) capable de fournir de l'énergie à l'un ou aux plusieurs capteurs conformément aux plages de consommation d'énergie, et le circuit de traitement (140) sont compris dans une étiquette pouvant être fixée au sujet.

6. Système de surveillance (100) selon la revendication 1, ou procédé de surveillance selon la revendication 2, dans lequel le mécanisme de détection (110) et une source d'énergie (130) capable de fournir de l'énergie à l'un ou aux plusieurs capteurs conformément aux plages de consommation d'énergie sont compris dans une étiquette pouvant être fixée au sujet.

7. Système de surveillance (100) selon la revendication 6, ou procédé de surveillance selon la revendication 6, dans lequel le circuit de traitement (140) fait partie d'un serveur, l'étiquette comprend en outre un émetteur-récepteur capable de transmettre les informations au serveur, et le procédé comprend en outre la réception des premières mesures et des deuxièmes mesures de l'étiquette à l'aide d'un émetteur.

8. Système de surveillance (100) selon la revendication 1, ou procédé de surveillance selon la revendication 2, dans lequel les un ou plusieurs capteurs incluent un ou plusieurs parmi : un capteur de vibrations, un capteur de température, un capteur de vitesse, un capteur d'accélération, un gyroscope, un magnétomètre, un podomètre, un capteur de localisation, un capteur de fréquence cardiaque, un capteur d'humidité.

9. Système de surveillance (100) selon la revendication 1, ou procédé de surveillance selon la revendication 2, dans lequel le sujet est un animal.

10. Système de surveillance (100) selon la revendication 1, ou procédé de surveillance selon la revendication 2, dans lequel les informations obtenues par au moins un des un ou plusieurs capteurs incluent une ou plusieurs parmi : (a) des informations physiologiques obtenues du sujet, (b) des informations géospatiales ou (c) des informations environnementales.

11. Support de stockage non transitoire lisible par ordinateur contenant un code de programme lisible par ordinateur, le code de programme lisible par ordinateur étant exécutable par au moins un circuit de traitement (140) d'un ordinateur pour effectuer un procédé comprenant les étapes consistant à :
provoquer, par le circuit de traitement (140), l'activation d'un mécanisme de détection (110) comprenant un ou plusieurs capteurs configurés pour obtenir des informations relatives à un sujet dans un premier mode de fonctionnement d'une pluralité de modes de fonctionnement du mécanisme de détection (110), pour obtenir une pluralité de premières mesures à partir des capteurs sur une première période de temps, dans lequel chacun des modes de fonctionnement présente des plages de consommation d'énergie respectives ;
analyser, par le circuit de traitement (140), les premières mesures pour déterminer un état comportemental du sujet, l'état comportemental étant l'un d'une pluralité d'états comportementaux prédéterminés, dans lequel (a) chaque état comportemental des états comportementaux prédéterminés est associé à un groupe distinct d'une pluralité d'états sous-comportementaux prédéterminés, et (b) dans au moins un de la pluralité d'états comportementaux prédéterminés, au moins une partie de ladite pluralité d'états sous-comportementaux prédéterminés sont des degrés dudit état comportemental ;
sur la base de l'état comportemental, provoquer l'activation, par le circuit de traitement (140), du mécanisme de détection (110) dans un deuxième mode de fonctionnement des modes de fonctionnement pour obtenir une pluralité de deuxièmes mesures à partir des un ou plusieurs capteurs sur une deuxième période de temps, le deuxième mode de fonctionnement présentant une plage de consommation d'énergie plus élevée que le premier mode de fonctionnement ;
analyser, par le circuit de traitement (140), les deuxièmes mesures pour déterminer un premier état sous-comportemental du sujet, le premier état sous-comportemental étant l'un des états sous-comportementaux du groupe des états sous-comportementaux prédéterminés associés à l'état comportemental ;
provoquer, par le circuit de traitement (140), après avoir déterminé l'état sous-comportemental du sujet, l'activation du mécanisme de détection (110) dans le premier mode de fonctionnement des modes de fonctionnement pour obtenir une pluralité de troisièmes mesures à partir des un ou plusieurs capteurs sur une troisième période de temps ;
analyser, par le circuit de traitement (140), les troisièmes mesures pour déterminer un deuxième état comportemental du sujet, le deuxième état comportemental étant l'un de la pluralité d'états comportementaux prédéterminés ;
sur la base du deuxième état comportemental, provoquer, par le circuit de traitement (140), l'activation du mécanisme de détection (110) dans un troisième mode de fonctionnement des modes de fonctionnement afin d'obtenir une pluralité de quatrièmes mesures à partir des un ou plusieurs capteurs sur une quatrième période de temps, le troisième mode de fonctionnement présentant une plage de consommation d'énergie supérieure à celle du premier mode de fonctionnement, dans lequel si le deuxième état comportemental est identique à l'état comportemental, l'activation du mécanisme de détection (110) dans le troisième mode de fonctionnement est effectuée lorsqu'un temps écoulé entre la détermination du deuxième état comportemental et une parmi (a) la détermination de l'état comportemental ou (b) la détermination du premier état sous-comportemental dépasse un seuil ; et
analyser, par le circuit de traitement (140), les quatrièmes mesures pour déterminer un deuxième état sous-comportemental du sujet, le deuxième état sous-comportemental étant l'un des états sous-comportementaux du groupe des états sous-comportementaux prédéterminés associés au deuxième état comportemental.
